# EUROPÄISCHE PATENTANMELDUNG

(11) **EP 4 464 769 A2**
(43) Veröffentlichungstag der Anmeldung: **20.11.2024**
(21) Anmeldenummer: 24204082.2
(22) Anmeldetag: 29.04.2019
(51) Int. Cl.: C12M 1/00

(54) **FILTERSYSTEM FÜR BIOPHARMAZEUTISCHE PROZESSE**

(30) Priorität: 19.06.2018 DE 102018004890
(62) Teilanmeldung aus: 19722036.1
(71) Anmelder: Sartorius Stedim Biotech GmbH, 37079 Göttingen (DE)
(72) Erfinder: MANZKE, Christian, 37120 Bovenden (DE)
(74) Vertreter: Müller-Boré & Partner Patentanwälte PartG mbB

(57) **Zusammenfassung**

Die vorliegende Erfindung betrifft ein Filtersystem für biopharmazeutische Prozesse umfassend:
- zumindest eine Filtereinheit; und
- zumindest eine Verteilerplatte, an welcher die zumindest eine Filtereinheit anliegt und welche eine Verteilereinheit umfasst, welche innerhalb der Verteilerplatte angeordnet ist und fluidisch mit der zumindest einen Filtereinheit verbunden ist;

wobei die Verteilereinheit ein Rohrleitungssystem umfasst, welches sich innerhalb der Verteilerplatte erstreckt und derart ausgebildet ist, dass das zu filtrierende Fluid über einen Eingangsanschluss der zumindest einen Filtereinheit zugeführt werden kann und/oder das filtrierte Fluid von der zumindest einen Filtereinheit aufgenommen und abgeführt werden kann, und
wobei die Verteilereinheit zumindest ein aktives Steuerelement umfasst, welches ausgebildet ist, einen Fluidstrom, welcher durch die Verteilereinheit und die zumindest eine Filtereinheit strömt, zu steuern oder zu regeln,
wobei die Verteilereinheit schlauchlos ist,
wobei die Fluidverbindung zwischen der zumindest einen Filtereinheit und der Verteilerplatte schlauchlos ist, und
wobei das Rohrleitungssystem zwei Ausganganschlüsse aufweist, über die das filtrierte Fluid aus der Verteilerplatte abgeführt werden kann.

## Beschreibung

Die vorliegende Erfindung betrifft ein Filtersystem für biopharmazeutische Prozesse, eine Verteilerplatte für biopharmazeutische Prozesse und ein Verfahren zum Herstellen eines Filtersystems für biopharmazeutische Prozesse.

Aus der Biopharmazie sind Prozesse wie beispielsweise Zellabtrennung (beispielsweise durch Tiefenfiltration), Sterilfiltration, Chromatographieschritte, Virusinaktivierung, Virusfiltration und/oder Crossflow-Filtration bekannt. Alle diese Prozesse stellen Grundoperationen dar, die innerhalb einer Filtereinheit durchgeführt werden können. Es ist außerdem bekannt, mehrere Filtereinheiten miteinander zu verschalten. Das zu filtrierende Fluid strömt dann zumindest teilweise und in Abhängigkeit der gewählten Prozesse sukzessive durch die einzelnen verschalteten Filtereinheiten.

Um das zu filtrierende Fluid zu einer Filtereinheit zu führen und ebenfalls von dieser abzuführen, werden Schläuche verwendet, die sowohl eine sterile als auch eine unsterile Übertragung ermöglichen. Insbesondere der Zuführungsschlauch, über den das zu filtrierende Fluid zu der Filtereinheit strömen kann, ist mit einer Pumpe zu verbinden, die das zu filtrierende Fluid durch die Filtereinheit pumpt. Darüber hinaus müssen weitere Elemente, wie beispielsweise Sensoren und Ventile, in die Schläuche integriert sein, um die Zufuhr bzw. Abfuhr des Fluids zu bzw. von der Filtereinheit weg zu regeln oder den Fluidstrom bzw. das Fluid zu überwachen.

Die Verwendung von Schläuchen führt dabei zu technischen Herausforderungen, da verschiedene Prozesse (wie z.B. Crossflowfiltration oder Virusfiltrationsapplikationen) unter hohem Druck (bis zu 6 bar) ablaufen können und somit eine Prozesssicherheit gewährleistet werden muss, die die Gefahr des Platzens oder Abreißens der Schläuche minimiert.

Weiterhin ist das Verbinden der verschiedenen Schläuche mit der Filtereinheit aufwendig, zeitintensiv und fehleranfällig. Insbesondere muss sichergestellt sein, dass bezüglich steriler Vorrichtungen die Schlauchverbindung steril ist. Darüber hinaus kann das Filtersystem nach Anschluss der Schlauchverbindung nicht sofort verwendet werden, da das gesamte System erst gespült und sterilisiert werden muss.

Es ist somit Aufgabe der vorliegenden Erfindung, die Handhabung eines Filtersystems zu vereinfachen und einen schnellen Einsatz des Filtersystems zu erlauben. Insbesondere hat das Filtersystem ein hohes Maß an Prozesssicherheit zu gewährleisten, so dass der Betreiber als auch das Produkt geschützt ist.

Diese Aufgabe wird durch ein Filtersystem für biopharmazeutische Prozesse gelöst, welche umfasst:
- zumindest eine Filtereinheit; und
- zumindest eine Verteilerplatte, an welcher die zumindest eine Filtereinheit anliegt und welche eine Verteilereinheit umfasst, welche innerhalb der Verteilerplatte angeordnet ist und fluidisch mit der zumindest einen Filtereinheit verbunden ist;
   wobei die Verteilereinheit dazu ausgelegt ist, das zu filtrierende Fluid zu der zumindest einen Filtereinheit zu führen und/oder das filtrierte Fluid von der zumindest einen Filtereinheit aufzunehmen und abzuführen, und
   wobei die Verteilereinheit zumindest ein aktives Steuerelement umfasst, mittels dem ein Fluidstrom, welcher durch die Verteilereinheit und die zumindest eine Filtereinheit strömt, steuerbar oder regelbar ist,
   wobei die Verteilereinheit schlauchlos ist, und
   wobei die Fluidverbindung zwischen der zumindest einen Filtereinheit und der Verteilerplatte schlauchlos ist.

Als "Filtereinheit" wird insbesondere eine Einheit verstanden, innerhalb der ein spezieller Prozessschritt für das gewünschte Verfahren ausgeführt wird. Insbesondere erfolgt innerhalb einer Filtereinheit eine Trennung von Komponenten eines Fluidstroms. Beispielsweise kann eine Filtereinheit eine Einheit zur Zellabtrennung (beispielsweise durch Tiefenfiltration), zur Sterilfiltration, für einen Chromatographieschritt, zur Virusabreicherung oder zur Crossflow-Filtration sein. Die Filtereinheit kann hier wie auch im Stand der Technik als Standard-Filtereinheit mit den gewünschten Spezifikationen ausgewählt werden. Umfasst das Filtersystem mehrere Filtereinheiten, können diese identisch ausgebildet sein oder zumindest teilweise unterschiedliche Eigenschaften aufweisen. Es können unterschiedliche Filtereinheiten miteinander verschaltet werden, die beispielsweise unterschiedliche Trennmedien verwenden oder für unterschiedliche Prozessierstufen eines Verfahrens zuständig sind. Gleiche Filtereinheiten können dann zum Einsatz kommen, wenn eine Kapazitätserweiterung gewünscht wird. Bei der Verschaltung der Filtereinheiten handelt es sich vorzugsweise um eine parallele Verschaltung der Filtereinheiten, wenn es sich um identische Filtereinheiten handelt. Eine serielle Verschaltung ist dann bevorzugt, wenn beispielsweise verschiedene Filtereinheiten miteinander verschaltet sind, die unterschiedliche Prozessierschritte durchführen.

Das "Filtersystem" kann mehrfach verwendet werden, ist jedoch insbesondere dazu geeignet, nur einmalig verwendet zu werden, so dass das Filtersystem nach seiner Verwendung entsorgt werden kann. Aufgrund der geringen Anzahl der Teile des erfindungsgemäßen Filtersystems ist das Filtersystem kostengünstig herzustellen ist und kann somit als Einweg-System genutzt werden.

Das Filtersystem kann als bereits montiertes bzw. vorkonfiguriertes und/oder steriles System an den Benutzer versendet werden, so dass das Filtersystem durch den Benutzer nur noch mit seinem bestehenden System zu verbinden ist (beispielsweise mit einem Behälter, in dem das zu filtrierende Fluid enthalten ist, und/oder Aktuatoren, wie z.B. einem Pumpenantrieb). Für den Benutzer ist es in diesem Fall insbesondere nicht mehr notwendig Spülungen oder ein Sterilisationsverfahren an dem Filtersystem (z.B. für Crossflowmembranen notwendig) durchzuführen. Dies kann bereits werkseitig vor Auslieferung des Filtersystems erfolgen. Das Filtersystem vereinfacht somit die Handhabung für den Benutzer und spart diesem Zeit.

Durch die direkte schlauchlose Verbindung zwischen der Verteilerplatte und der Filtereinheit, können jegliche Schläuche bzw. flexible Verbindungen zwischen diesen beiden Elementen vermieden werden. Darüber hinaus ist die Verteilereinheit in die Verteilerplatte integriert, so dass die Verteilerplatte ebenfalls jegliche Schläuche bzw. flexible Verbindungen einspart. Durch die Vermeidung dieser Schlauchverbindungen können kritische Stellen vermieden werden, an denen ein Risiko für die Integrität und/oder Sterilität des Systems besteht. Insbesondere da das System als bereits montiertes und steriles System ausgeliefert werden kann, kann das Risiko vermieden werden, dass dem Benutzer Fehler in der Montage unterlaufen und infolgedessen kein abgeschlossenes und/oder steriles Filtersystem gewährleistet wird.

Durch die Vermeidung von Schläuchen kann insbesondere auch vermieden werden, dass die Druckbeständigkeit der Schläuche ständig gewährleistet sein muss bzw. überwacht werden muss. Darüber hinaus ist es nicht mehr notwendig, Messstellen in den Schläuchen bereitzustellen, die üblicherweise nur in Abschnitte integriert sein konnten, wo der Schlauch einen entsprechenden Durchmesser aufweist.

Zudem weist die Verteilereinheit zumindest ein aktives Steuerelement auf, mittels dem der Fluidstrom durch die Verteilereinheit als auch durch die zumindest eine Filtereinheit geregelt oder gesteuert werden kann. Dies bietet weiterhin den Vorteil, dass notwendige Komponenten zum Betreiben des Filtersystems vormontiert sind, so dass auch hier die Handhabung für den Benutzer verbessert wird.

Vorzugsweise umfasst die Verteilereinheit ein Rohrleitungssystem, welches sich innerhalb der Verteilerplatte erstreckt und dazu ausgelegt ist, das zu filtrierende Fluid und/oder das filtrierte Fluid zu führen.

Über das Rohrleitungssystem kann das zu filtrierende Fluid der zumindest einen Filtereinheit zugeführt werden und das filtrierte Fluid ("Filtrat") von der Filtereinheit abgeführt werden.

Mit anderen Worten hat das Rohrleitungssystem vorzugsweise zumindest einen Zuführ- bzw. Speisezugang bzw. Eingangsanschluss, über den das zu filtrierende Fluid in das Rohrleitungssystem eintritt und anschließend der zumindest einen Filtereinheit zugeführt wird. Weiterhin hat das Rohrleitungssystem vorzugsweise zumindest einen Abführausgang bzw. Ausgangsanschluss, über den das filtrierte Fluid aus der Verteilerplatte abgeführt werden kann. Der Benutzer muss somit vor Inbetriebnahme des Filtersystems in entsprechender Weise an die Anschlüsse einen Tank mit dem zu filtrierenden Fluid sowie einen Auffangbehälter mit dem filtrierten Fluid an die Verteilerplatte anschließen. Vorzugsweise handelt es sich bei den Anschlüssen um Sterilkonnektoren.

Da das Rohrleitungssystem in die Verteilerplatte integriert ist bzw. in die Verteilerplatte eingearbeitet ist, bestehen innerhalb dieses Rohrleitungssystems keine Kontaktstellen zwischen einzelnen Schlauchelementen, die die Sterilität des Filtersystems gefährden könnten. Es können jegliche Schläuche zur Führung des Fluidstroms vermieden werden.

Es ist ferner bevorzugt, dass das aktive Steuerelement zumindest einen Pumpenkopf umfasst, der mit einem Pumpenantrieb verbindbar ist, um das Fluid durch die Verteilerplatte und die zumindest eine Filtereinheit zu pumpen.

Mit anderen Worten kann der relativ kostengünstige Pumpenkopf in die Verteilerplatte integriert bzw. in diese eingearbeitet sein. Der Pumpenkopf selbst kann vor Inbetriebnahme des Filtersystems dann in einfacher Weise mit einem Pumpenantrieb des Benutzers verbunden werden. Dies kann beispielsweise durch ein einfaches Aufstecken des Pumpenkopfs auf den Pumpenantrieb erfolgen. Eine derartige Konfiguration erlaubt insbesondere auch eine Einweg-Verwendung des Filtersystems.

Die Pumpe dient dazu, das zu filtrierende Fluid mit einem entsprechenden Druck durch die zumindest eine Filtereinheit zu pressen. Der aufzubringende Druck des Fluidstroms richtet sich dabei insbesondere nach der verwendeten Filtereinheit.

Durch die genannte Verbindungsweise des Pumpenantriebs mit der Verteilerplatte, kann der Pumpenantrieb ohne jegliche Schläuche und in einfacher Weise an das Filtersystem angeschlossen werden. Die Handhabung für den Benutzer wird hierbei deutlich verbessert.

Da der Pumpenantrieb normalerweise nicht sterilisierbar ist, kann der Pumpenantrieb durch eine Membran bzw. ein Septum oder eine entsprechende mechanische Kupplung von dem Fluid abgetrennt sein. Die Membran oder mechanische Kupplung selbst kann in die Verteilerplatte integriert sein und bereits werkseitig als Teil des Filtersystems sterilisiert werden.

Vorzugsweise umfasst das aktive Steuerelement zumindest ein Ventil, welches dazu ausgelegt ist, den Fluidstrom durch die Verteilerplatte und/oder die zumindest eine Filtereinheit zu regeln oder zu steuern.

Das zumindest eine Ventil ist vorzugsweise in das Rohrleitungssystem integriert. Das zumindest eine Ventil kann dabei dazu ausgelegt sein, den Fluidstrom durch die Verteilerplatte zu regeln oder zu steuern. Das zumindest eine Ventil kann den notwendigen Staudruck aufbauen, um das Fluid mit dem erforderlichen Druck durch die Filtermembran zu pressen. Weiterhin kann das Ventil beispielsweise dazu genutzt werden, den Fluidstrom nur in eine Richtung des Rohrleitungssystems strömen zu lassen, und/oder die Durchflussmenge durch das Rohrleitungssystem zu regeln oder zu steuern. Weiterhin kann ein Ventil an einem Speisezugang an der Verteilerplatte angeordnet sein, um den Zufluss in die Verteilerplatte zu steuern oder zu regeln.

Das Ventil kann händisch, pneumatisch oder elektrisch betrieben werden. Insbesondere kann das Ventil mit einer Steuereinrichtung des Benutzers verbunden werden, um über die Steuereinrichtung das Ventil zu steuern.

Es ist ferner bevorzugt, dass die Verteilereinheit zumindest einen Sensor umfasst, welcher dazu ausgelegt ist, zumindest einen Parameter des Fluidstroms innerhalb der Verteilereinheit zu messen.

Vorzugsweise handelt es sich bei dem Sensor um einen Drucksensor, welcher in die Verteilerplatte integriert ist. Der Drucksensor kann dabei so angeordnet sein, dass dieser innerhalb des Rohrleitungssystems der Verteilerplatte den Druck des Fluidstroms misst bzw. überwacht. Hierdurch kann gewährleistet werden, dass das zu filtrierende Fluid mit dem notwendigen Druck auf die Filtereinheit auftrifft. Hierzu kann der Drucksensor mit der Steuereinrichtung des Benutzers verbunden werden. Anhand der gemessenen Werte kann dann entweder über die Pumpe oder die oben genannten Ventile der Druck geregelt werden.

Beispielsweise kann mittels dem zumindest einen Sensor der Druck, der Volumenstrom, der UV-Wert, der pH-Wert, die Trübung und/oder die Viskosität des Fluids gemessen werden. Weiterhin ist es möglich, die Leitfähigkeit, den volumetrischen Fluss und/oder die UV-Absorption zu messen.

Vorzugsweise umfasst das Filtersystem ferner zumindest eine Abschlussplatte, welche mittels zumindest einem Halteelement mit der Verteilerplatte verbunden ist,
wobei die Filtereinheit zwischen der Verteilerplatte und der Abschlussplatte angeordnet ist und durch die Abschlussplatte an der Verteilerplatte gehalten wird.

Mit anderen Worten kann die zumindest eine Filtereinheit zwischen die Verteilerplatte und die Abschlussplatte geklemmt werden. Das zumindest eine Halteelement dient als Verbindungselement zwischen der Verteilerplatte und der Abschlussplatte und ist dazu ausgelegt, die notwendige Haltekraft für die zumindest eine Filtereinheit aufzubringen.

Vorzugsweise handelt es sich bei dem Halteelement um eine Haltestange, welche mittels einer Schraubverbindung mit der Abschlussplatte und der Verteilerplatte verbunden sein kann.

Die Abschlussplatte ist insbesondere für Ausführungen eines Filtersystems vorteilhaft, in der mehr als eine Filtereinheit an der Verteilerplatte angeordnet ist. Durch die Abschlussplatte kann die notwendige Haltekraft aufgebracht werden, um die Filtereinheiten an der Verteilerplatte zu halten.

Weiterhin ist es bevorzugt, dass die Verteilerplatte aus Kunststoff gefertigt ist.

Aufgrund einer Fertigung der Verteilerplatte aus Kunststoff kann diese kostengünstig hergestellt werden, so dass sich die Verteilerplatte für eine Einweg-Verwendung eignet. Dies kann beispielsweise mittels eines Spritzgießverfahrens, 3D-Drucks oder CNC-Fräsverfahrens erfolgen.

In einer bevorzugten Ausführungsform ist die zumindest eine Filtereinheit und/oder die Verteilerplatte durch Gamma-Bestrahlung, Begasung und/oder Autoklavieren sterilisierbar ist. In einer besonders bevorzugten Ausführungsform ist die zumindest eine Filtereinheit und/oder die Verteilerplatte durch Gamma-Bestrahlung sterilisierbar.

Sind die Filtereinheit und die Verteilerplatte mittels der genannten Methoden sterilisierbar, kann das gesamte Filtersystem als eine Einheit sterilisiert werden und anschließend als steriles Produkt an den Benutzer ausgeliefert werden. Der Benutzer selbst hat vor Benutzung des Systems keine Sterilisationsmaßnahmen mehr durchzuführen. Vorzugsweise werden hierfür Sterilkonnektoren als Anschlusselemente an der Verteilerplatte verwendet, die dazu dienen, das zu filtrierende Fluid der Verteilerplatte zuzuführen und das filtrierte Fluid von der Verteilerplatte abzuführen.

Gamma-Bestrahlung bietet hierbei beispielsweise den Vorteil, dass in billiger und einfacher Weise eine Sterilisation des Filtersystems vorgenommen werden kann und das Filtersystem keiner hohen thermischen Belastung ausgesetzt ist.

Vorzugsweise umfasst das Filtersystem zumindest eine erste Filtereinheit und zumindest eine zweite Filtereinheit, wobei die zumindest eine erste und die zumindest eine zweite Filtereinheit auf entgegengesetzten Seiten der Verteilerplatte anliegen.

Durch die Anordnung von Filtereinheiten auf entgegengesetzten Seiten der Verteilerplatte kann die zu nutzende Filterfläche in einfacher Weise vergrößert werden.

Vorzugsweise sind die Filtereinheit und die Verteilerplatte miteinander verklebt oder aus einem Stück spritzgegossen.

Gemäß einem weiteren Aspekt der vorliegenden Erfindung wird die vorliegende technische Aufgabe durch eine Verteilerplatte für ein Filtersystem für biopharmazeutische Prozesse gelöst, welche eine Verteilereinheit umfasst, welche innerhalb der Verteilerplatte angeordnet ist und fluidisch mit zumindest einer Filtereinheit verbindbar ist, indem die Filtereinheit an der Verteilerplatte anliegt;
wobei die Verteilereinheit dazu ausgelegt ist, das zu filtrierende Fluid zu der zumindest einen Filtereinheit zu führen und/oder das filtrierte Fluid von der zumindest einen Filtereinheit aufzunehmen und abzuführen, und
wobei die Verteilereinheit zumindest ein aktives Steuerelement umfasst, mittels dem ein Fluidstrom, welcher durch die Verteilereinheit und die zumindest eine Filtereinheit strömt, steuerbar oder regelbar ist,
wobei die Verteilereinheit schlauchlos ist, und
wobei die Fluidverbindung zwischen der zumindest einen Filtereinheit und der Verteilerplatte schlauchlos ist.

Gemäß einem weiteren Aspekt der vorliegenden Erfindung wird die vorliegende technische Aufgabe durch ein Verfahren zum Herstellen eines Filtersystems für biopharmazeutische Prozesse gelöst, welche die Schritte umfasst:
- Bereitstellen zumindest einer Filtereinheit; und
- Bereitstellen zumindest einer Verteilerplatte, welche eine schlauchlose Verteilereinheit, welche innerhalb der Verteilerplatte angeordnet ist, und zumindest ein aktives Steuerelement umfasst; und
- Schlauchloses Anordnen der zumindest einen Filtereinheit an der Verteilerplatte, derart dass die zumindest eine Filtereinheit und die Verteilereinheit fluidisch miteinander verbunden sind;
   wobei die Verteilereinheit dazu ausgelegt ist, das zu filtrierende Fluid zu der zumindest einen Filtereinheit zu führen und/oder das filtrierte Fluid von der zumindest einen Filtereinheit aufzunehmen und abzuführen, und
   wobei das zumindest eine aktive Steuerelement dazu ausgelegt ist, einen Fluidstrom, welcher durch die Verteilereinheit und die zumindest eine Filtereinheit strömt, zu steuern oder zu regeln.

Diese und andere Ziele, Merkmale und Vorteile der vorliegenden Erfindung werden durch das Studium der folgenden detaillierten Beschreibung bevorzugter Ausführungsformen und der beigefügten Zeichnungen klarer. Weiterhin wird darauf hingewiesen, dass, obwohl Ausführungsformen separat beschrieben werden, einzelne Merkmale dieser Ausführungsformen zu zusätzlichen Ausführungsformen kombiniert werden können.
- Fig. 1: Draufsicht auf ein Filtersystem mit einer Filtereinheit;
- Fig. 2: Draufsicht auf ein Filtersystem mit zwei Filtereinheiten, die auf entgegengesetzten Seiten einer Verteilerplatte angeordnet sind;
- Fig. 3: zeigt eine Seitenansicht der Verteilerplatte;
- Fig. 4: zeigt eine Explosionsansicht einer Filtereinheit für Crossflow-Filtration;
- Fig. 5a) und 5b): zeigen verschiedene Montagearten des Filtersystems an einem Arbeitstisch;
- Fig. 6: zeigt eine weitere Ausführungsform eines Filtersystems mit vier Filtereinheiten.

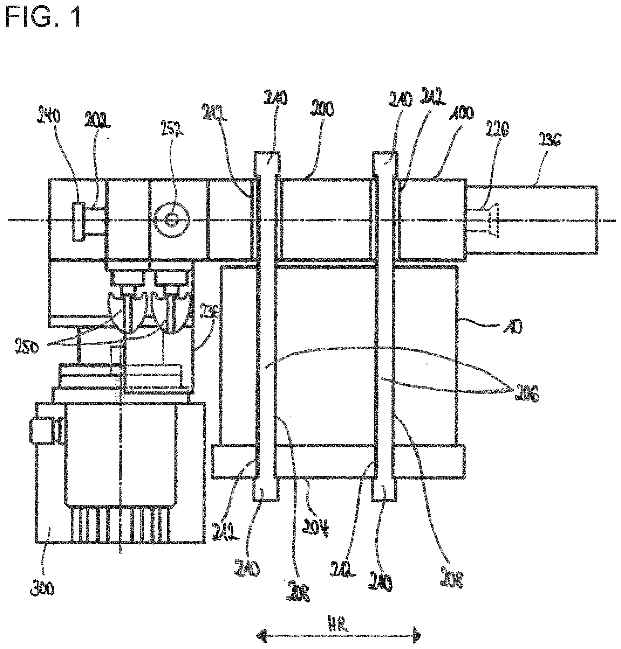

Das erfindungsgemäße Filtersystem 100 für biopharmazeutische Prozesse umfasst eine oder mehrere Filtereinheiten 10. Die Filtereinheiten 10 können Einweg-Filtereinheiten, die zur einmaligen Nutzung ausgelegt sind, oder Mehrweg-Filtereinheiten sein. **Figur 1** zeigt eine Draufsicht auf das Filtersystem 100 mit einer Filtereinheit 10. Es können jedoch auch mehrere Filtereinheiten 10 seriell oder parallel miteinander in dem Filtersystem 100 verschaltet sein. Das im Folgenden beschriebene Filtersystem 100 ist insbesondere zur Crossflowfiltration ausgelegt. Das Filtersystem 100 kann jedoch auch für andere Filtrationsmethoden verwendet werden. Je nach verwendeten Filtereinheiten 10 bzw. Filterarten ist hierbei die Fluidführung in der unten beschriebenen Verteilerplatte angepasst.

Zusätzlich zu der bereits erwähnten zumindest einen Filtereinheit 10 umfasst das Filtersystem 100 eine Verteilerplatte 200, an der die zumindest eine Filtereinheit 10 anliegt. Die Verteilerplatte 200 kann als Einweg-Verteilerplatte oder als Mehrweg-Verteilerplatte ausgebildet sein. Insbesondere kann die Verteilerplatte 200 aus Kunststoff gefertigt sein, wodurch sie sich in vorteilhafter Weise als Einweg-Element eignet. Vorzugsweise ist die Verteilerplatte 200 spritzgegossen. Die zumindest eine Filtereinheit 10 ist dabei derart an der Verteilerplatte 200 angeordnet, dass sich zumindest ein Filtermedium (hier nicht gezeigt), welches sich in der Filtereinheit 10 befindet, im Wesentlichen parallel zu der Verteilerplatte 200 erstreckt.

In die Verteilerplatte 200 ist eine Verteilereinheit 202 integriert, die dazu ausgelegt ist, in Fluidverbindung mit der zumindest einen Filtereinheit 10 zu stehen.

Die Verteilerplatte 200 kann mit der Filtereinheit 10 (bei mehreren verschalteten Filtereinheiten 10 die Filtereinheit 10, die direkt an der Verteilerplatte 200 anliegt) verklebt sein oder zusammen mit der zumindest einen Filtereinheit 10 spritzgegossen werden. Weiterhin können benachbarte Filtereinheiten 10 ebenfalls miteinander verklebt sein oder zusammen spritzgegossen sein. Wie in Figur 1 gezeigt, kann die zumindest eine Filtereinheit 10 alternativ oder ergänzend mit zumindest einer Abschlussplatte 204 und zumindest einem Halteelement 206 an der Verteilerplatte 200 gehalten werden. Die zumindest eine Filtereinheit 10 wird hierbei zwischen der Verteilerplatte 200 und der Abschlussplatte 204 angeordnet. Die Abschlussplatte 204 und die Verteilerplatte 200 sind mittels des zumindest einen Halteelements 206 miteinander verbunden. Wie in Figur 1 gezeigt, kann das Halteelement 206 als Haltestange 208 ausgebildet sein bzw. zumindest eine Haltestange 208 umfassen. In Figur 1 wird die Abschlussplatte 204 und die Verteilerplatte 200 mittels vier Haltestangen 208 verbunden, wobei aufgrund der verwendeten Perspektive lediglich zwei Haltestangen 208 sichtbar sind.

Die Haltestangen 208 können die Verteilerplatte 200 und die Abschlussplatte 204 mit Hilfe von Durchgangsbohrungen durchdringen, wobei Enden der Haltestangen 208 jeweils über die Verteilerplatte 200 und die Abschlussplatte 204 hinausragen. Auf diese Enden ist jeweils zumindest eine Abschlussmutter 210 aufgeschraubt. Diese verhindert das Herausrutschen der Haltestange 208 und erlaubt durch deren Position an der Haltestange 208 das Einstellen des nötigen Drucks auf die zumindest eine Filtereinheit 10, um diese sicher zwischen der Verteilerplatte 200 und der Abschlussplatte 204 zu halten.

Alternativ können, wie in Figur 1 gezeigt, Kerben bzw. Ausnehmungen 212 in einer Oberseite bzw. Unterseite (hier nicht sichtbar) der Verteilerplatte 200 und der Abschlussplatte 204 ausgebildet sein, in die die Haltestangen 208 jeweils eingelegt werden. Die Haltestangen 208 sind auch hier, wie oben beschrieben, mit Abschlussmuttern 210 gesichert.

Die Abschlussplatte 204 sowie die Halteelemente 206 können aus Kunststoff oder Metall gefertigt sein.

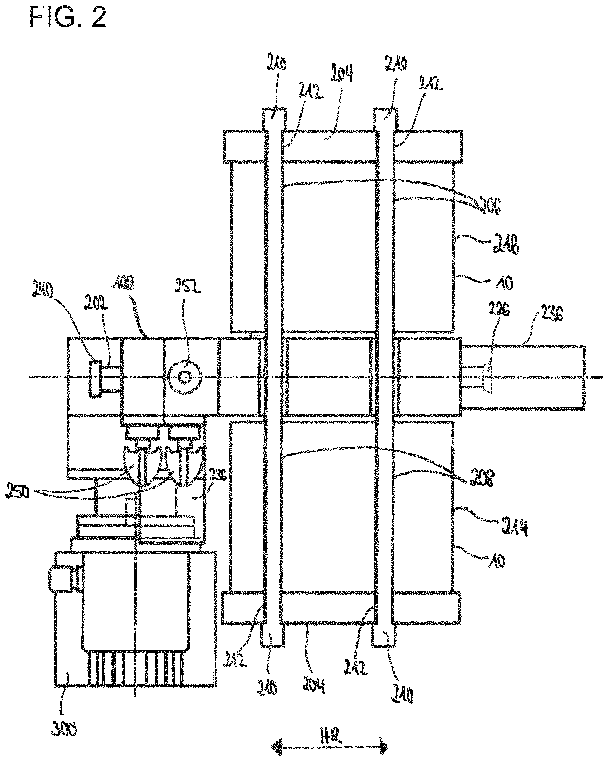

**Figur 2** zeigt eine weitere Ausführungsform eines Filtersystems 100. Das Filtersystem 100 in Figur 2 unterscheidet sich von dem Filtersystem 100 aus Figur 1 darin, dass hier zumindest eine weitere Filtereinheit 10 an der Verteilerplatte 200 anliegt. Es werden daher im Folgenden nur die sich unterscheidenden Merkmale beschrieben. Alle übrigen Definitionen und Beschreibungen zu Figur 1 gelten auch für Figur 2.

Wie in Figur 2 gezeigt, umfasst das Filtersystem 100 hier eine erste Filtereinheit 214 und eine zweite Filtereinheit 216, die an entgegengesetzten Seiten der Verteilerplatte 200 anliegen. Obwohl in Figur 2 einzelne Filtereinheiten 10 auf den entgegengesetzten Seiten der Verteilerplatte 200 gezeigt sind, können auf jeder der Seiten auch mehrere Filtereinheiten 10 angeordnet sein, die parallel oder seriell miteinander verschaltet sind. Dies erfolgt in Abhängigkeit der benötigten Filterfläche des Filtersystems 100.

Die verwendeten Haltestangen 208 weisen vorzugsweise eine derartige Länge auf, dass diese dazu geeignet sind, sowohl die erste Filtereinheit 214 als auch die zweite Filtereinheit 216 an der Verteilerplatte 200 zu befestigen, wie in Figur 2 gezeigt. Es ist jedoch ebenfalls möglich, dass jede Filtereinheit 10 separat für sich durch entsprechende Halteelemente 206 bzw. Haltestangen 208 gesichert ist.

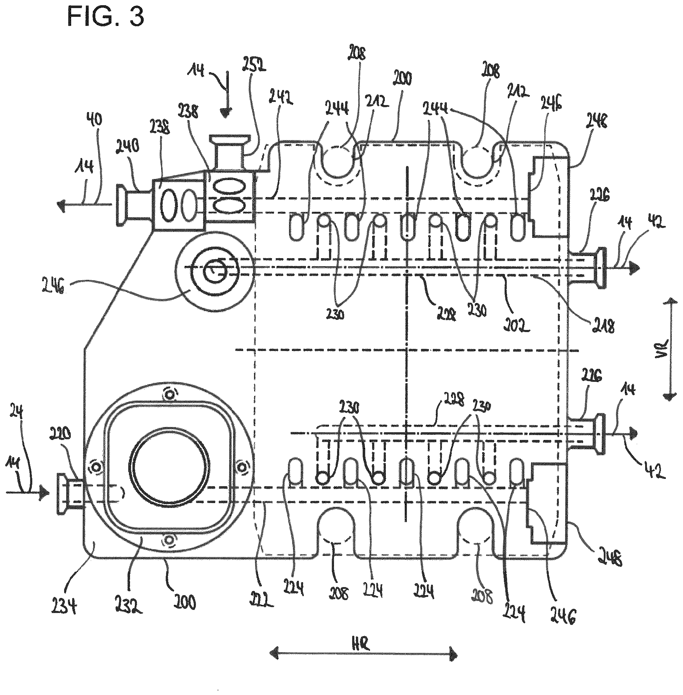

**Figur 3** zeigt eine Seitenansicht einer Verteilerplatte 200. Mit Hilfe unterbrochener Linien wird ein schlauchloses Rohrleitungssystem 218 dargestellt, welches in die Verteilerplatte 200 als Teil der Verteilereinheit 202 integriert ist. Das Rohrleitungssystem 218 erstreckt sich im Wesentlichen innerhalb der Verteilerplatte 200 bzw. ist in die Verteilerplatte 200 eingearbeitet. Es können somit jegliche Schläuche vermieden werden.

Über das Rohrleitungssystem 218 kann das zu filtrierende Medium zu der zumindest einen Filtereinheit 10 geführt werden. Nach Passieren der zumindest einen Filtereinheit 10 können dann die aufgetrennten Fluidströme 14 über das Rohrleitungssystem 218 in der Verteilerplatte 200 wieder abgeführt werden. Sowohl innerhalb der Verteilerplatte 200 als auch für die Fluidverbindung zwischen der Verteilerplatte 200 und der zumindest einen Filtereinheit 10 können jegliche Schläuche vermieden werden, so dass die Verteilereinheit 202 sowie die zumindest eine Filtereinheit 10 fluidisch schlauchlos miteinander verbunden sind.

Das Rohrleitungssystem 218 umfasst zumindest einen Eingangsanschluss 220, mittels dem das Filtersystem 100 mit einem zu filtrierenden Medium gespeist werden kann. An diesen Eingangsanschluss 220 kann das Filtersystem 100 mit zumindest einem Tank (hier nicht gezeigt) verbunden werden, in dem das zu filtrierende Medium gelagert ist. Wie in Figur 3 gezeigt ist, kann es sich bei dem Eingangsanschluss 220 um einen Anschlussstutzen handeln. Insbesondere kann es sich bei dem Eingangsanschluss 220 um einen Sterilkonnektor handeln. Der Eingangsanschluss 220 ist in Figur 3 an der linken Seite der Verteilerplatte 200 angeordnet. Vorzugsweise befindet sich der Eingangsanschluss 220 in Bezug auf eine vertikale Richtung VR an einem unteren Ende der Verteilerplatte 200.

Über den Eingangsanschluss 220 kann der Fluidstrom 14 in die Verteilerplatte 200 einströmen und anschließend in eine Zuführungsleitung 222 strömen. Die Zuführungsleitung 222 erstreckt sich dabei vorzugsweise in horizontaler Richtung HR und ist mit zumindest einer Einströmöffnung 224 verbunden, über die das zu filtrierende Fluid in die zumindest eine Filtereinheit 10 einströmen kann. Wie in Figur 3 gezeigt, können mehrere Einströmöffnungen 224 entlang der horizontalen Richtung HR angeordnet sein. Weiterhin können Einströmöffnungen 224 auf weiteren Ebenen (bezüglich der vertikalen Richtung VR) der Verteilerplatte 200 angeordnet sein, so dass das zu filtrierende Fluid auf mehreren Ebenen des Filtermediums in die Filtereinheit 10 einströmen kann. Die Einströmöffnungen 224 haben vorzugsweise einen schlitzförmigen Querschnitt mit vorzugsweise abgerundeten Ecken, können aber beispielsweise auch rund oder oval sein.

Auf der vorzugsweise entgegengesetzten Seite der Verteilerplatte 200, an der der Eingangsanschluss 220 angeordnet ist, ist vorzugsweise zumindest ein Permeat-Ausgangsanschluss 226 ausgebildet, welcher zum Abführen des Permeats aus der Verteilerplatte 200 dient. Dieser kann, wie auch der Eingangsanschluss 220, als Anschlussstutzen ausgebildet sein. Insbesondere kann der Permeat-Ausgangsanschluss 226 als Sterilkonnektor ausgebildet sein. Figur 3 zeigt zwei Permeat-Ausgangsanschlüsse 226, welche vorzugsweise in vertikaler Richtung VR versetzt angeordnet sind.

An die genannten Permeat-Ausgangsanschlüsse 226 kann jeweils beispielsweise ein Schlauch (hier nicht gezeigt) angeschlossen werden, über die das Permeat (also der Anteil des Fluidstroms 14, der das Filtermedium passiert hat) aus der Verteilerplatte 200 abgeführt werden kann. Die Permeat-Ausgangsanschlüsse 226 sind jeweils mit einer Permeat-Abführleitung 228 verbunden, welche sich vorzugsweise in horizontaler Richtung HR in der Verteilerplatte 200 erstreckt. Die Permeat-Abführleitung 228 ist mit zumindest einer Permeat-Abführöffnung 230 verbunden, über die das Permeat aus der zumindest einen Filtereinheit 10 in die Permeat-Abführleitung 228 einströmen kann. Wie in Figur 3 gezeigt, können mehrere Permeat-Abführöffnungen 230 entlang der einzelnen Permeat-Abführleitungen 228 angeordnet sein. Die Permeat-Abführöffnung 230 selbst hat hier einen kreisrunden Querschnitt, kann aber alternativ oval oder schlitzförmig sein. Einströmöffnungen 224 und Permeat-Abführöffnungen 230 können sich auf einer Ebene befinden und abwechselnd angeordnet sein.

Weiterhin umfasst das Filtersystem 100 zumindest einen Retentat-Ausgangsanschluss 240, über den das Retentat (also der Anteil des zugeführten Fluids, der das Filtermedium in der Filtereinheit 10 nicht passiert hat) aus der Verteilerplatte 200 abgeführt werden kann. Der Retentat-Ausgangsanschluss 240 kann ebenfalls als Anschlussstutzen und/oder als Sterilkonnektor ausgebildet sein. Wie in Figur 3 gezeigt, kann der Retentat-Ausgangsanschluss 240 auf derselben Seite der Verteilerplatte 200 angeordnet sein wie der Eingangsanschluss 220. Vorzugsweise ist der Retentat-Ausgangsanschluss 240 in Bezug auf die vertikale Richtung VR oberhalb des Eingangsanschlusses 220 angeordnet bzw. an einem oberen Ende der Verteilerplatte 200.

An den Retentat-Ausgangsanschluss 240 kann beispielsweise ein Schlauch (hier nicht gezeigt) angeschlossen werden, über den das Retentat aus der Verteilerplatte 200 abgeführt werden kann. Der Retentat-Ausgangsanschluss 240 ist mit einer Retentat-Abführleitung 242 verbunden, welche sich vorzugsweise in horizontaler Richtung HR in der Verteilerplatte 200 erstreckt. Die Retentat-Abführleitung 242 ist mit zumindest einer Retentat-Abführöffnung 244 verbunden, über die das Retentat aus der zumindest einen Filtereinheit 10 in die Retentat-Abführleitung 242 einströmen kann. Wie in Figur 3 gezeigt, können mehrere Retentat-Abführöffnungen 244 entlang der Retentat-Abführleitung 242 angeordnet sein. Die Retentat-Abführöffnung 244 kann ähnlich zu der Einströmöffnung 224 ausgebildet sein. Retentat-Abführöffnungen 244 und Permeat-Abführöffnungen 230 können sich auf einer Ebene befinden und abwechselnd angeordnet sein.

Sind an entgegengesetzten Seiten der Verteilerplatte 200 jeweils zumindest eine Filtereinheit 10 angeordnet, sind zumindest eine weitere Einströmöffnung 224, zumindest eine weitere Permeat-Abführöffnung 230 und zumindest eine weitere Retentat-Abführöffnung 244 auf einer nicht gezeigten Hinterseite der Verteilerplatte 200 angeordnet, die in entsprechender Weise die Zuführleitung 222, die Permeat-Abführleitung 228 und die Retentat-Abführleitung 242 mit der zumindest einen weiteren Filtereinheit 10 auf der Hinterseite der Verteilerplatte 200 verbindet.

Um einen Fluidstrom 14 durch das Filtersystem 100 zu gewährleisten und den notwendigen Druck aufzubringen, der erforderlich ist, um den Filtrationsprozess zu ermöglichen, wird ein Pumpe (in Figur 3 nicht gezeigt) benötigt. Diese ist an die Verteilerplatte 200 anschließbar. Um eine schnelle und einfache Anschlussmöglichkeit bereitzustellen, ist ein Pumpenkopf 232 als aktives Steuerelement und als Teil der Verteilereinheit 202 in die Verteilerplatte 200 integriert. Wird die Verteilerplatte 200 beispielsweise aus Kunststoff hergestellt, kann der Pumpenkopf 232 im Rahmen eines Spritzgießverfahrens in die Verteilerplatte 200 eingearbeitet werden. Da der Pumpenkopf 232 eine kostengünstige Komponente darstellt, kann das Filtersystem 100 hierdurch als Einweg-System verwendet werden, d.h. das Filtersystem 100 kann nach Gebrauch entsorgt werden. Der Pumpenantrieb 300 selbst kann wiederverwendet werden. Vorzugsweise ist die Pumpe bzw. der Pumpenkopf 232 derart in bzw. an der Verteilerplatte 200 angeordnet, dass die Pumpe mit der Zuführungsleitung 222 in Verbindung steht. Weiterhin kann der Pumpenkopf 232 ein Septum umfassen, so dass die Pumpe nicht mit dem Fluidstrom 14 in der Verteilerplatte 200 in Berührung kommt.

Der Pumpenantrieb 300 kann beispielsweise mit dem Pumpenkopf 232 über einen Schraubmechanismus oder mittels eines Klickverschlusses verbunden werden. Die Figuren 1 und 2 zeigen jeweils einen Zustand, in dem der Pumpenantrieb 300 bereits mit dem Filtersystem 100 verbunden ist bzw. an die Verteilerplatte 200 angeschlossen ist.

Ergänzend kann die Verteilereinheit 202 zumindest einen Sensor enthalten, der ebenfalls in die Verteilerplatte 200 integriert ist. Ein integrierter Sensor ist vorzugsweise ein Einweg-Sensor, wenn das Filtersystem als Einweg-System verwendet wird. Alternativ oder ergänzend kann zumindest ein Sensor 236 an die Verteilerplatte 200 angeschlossen werden und ist somit wiederverwendbar. Für diese Möglichkeit weist die Verteilerplatte 200 vorzugsweise eine integrierte Anschlussstelle auf.

Figur 3 zeigt eine Ausführungsform in der mehrere Sensoren 236 an die Verteilerplatte 200 anschließbar sind. Die Anschlussstelle kann vorzugsweise ein Septum 246 aufweisen. Dies ist in Figur 3 beispielsweise bezüglich der oberen Permeat-Abführleitung 228 gezeigt. Ein anschließbarer Sensor 236 würde hier derart anschließbar sein, dass der Fluidstrom 14 in der oberen Permeat-Abführleitung 228 messbar ist. Der anschließbare Sensor 236 wäre derart anschließbar, dass sich der Sensor 236 selbst aus der Bildebene heraus (also senkrecht zur horizontalen Richtung HR) erstrecken würde.

Weiterhin kann die Verteilerplatte 200 zumindest eine Anschlussausnehmung 248 aufweisen, die als Anschlussstelle für einen Sensor 236 dient. Figur 3 zeigt hier auf der rechten Seite der Verteilerplatte 200 jeweils eine Anschlussausnehmung 248, welche den Anschluss jeweils eines Sensors 236 an die Zuführungsleitung 222 und die Retentat-Abführleitung 242 erlaubt. Die Figuren 1 und 2 zeigen jeweils einen Zustand in dem ein Sensor 236 an bzw. in der Anschlussausnehmung 248 angeordnet ist bzw. ein Sensor 236 mit der Verteilerplatte 200 verbunden ist.

Jeder der genannten Sensoren 236 ist dabei an oder in dem Rohrleitungssystem 218 der Verteilerplatte 200 angeordnet, so dass der Sensor 236 in Kontakt mit dem Fluidstrom 14 durch die Verteilerplatte 200 steht. Hierdurch können verschiedene Parameter des Fluids überwacht werden. Der Sensor 236 selbst kann durch den Benutzer mit einer Steuereinrichtung zur Überwachung verbunden werden, die außerhalb des Filtersystems 100 angeordnet ist.

Bei dem Sensor 236 kann es sich um einen Drucksensor 236 handeln, der den Druck des Fluidstroms 14 überwacht. Sollte der Druck des Fluidstroms 14 von den vorgesehenen Werten abweichen, kann beispielsweise mit Hilfe der Pumpe, die ebenfalls mit der Steuereinrichtung verbunden sein kann, der Druck des Fluidstroms 14 geregelt werden.

Weiterhin kann als aktives Steuerelement und als Teil der Verteilereinheit 202 zumindest ein Ventil 238 in die Verteilerplatte 200 integriert sein. Das Ventil 238 ist vorzugsweise in oder an dem Rohrleitungssystem 218 angeordnet. Insbesondere ist es bevorzugt, dass das Ventil 238 an oder vor einem Retentat-Ausgangsanschluss 240 angeordnet ist, wie in Figur 3 gezeigt. Das Ventil 238 kann zum einen sicherstellen, dass der Fluidstrom 14 durch die Verteilerplatte 200 nur in eine Richtung erfolgen kann. Ein Rückwärtsströmen kann somit vermieden werden. Darüber hinaus kann durch ein Ventil 238 ein Weiterströmen, ein Einströmen oder ein Ausströmen des Fluidstroms 14 in die bzw. aus der Verteilerplatte 200 blockiert werden. Alternativ kann durch das Ventil 238 auch der Fluidstrom 14 selbst geregelt oder gesteuert werden. Je nach Position oder Öffnungsstellung des Ventils 238 kann beispielsweise der Druck des Fluidstroms 14 beeinflusst werden. Hierzu kann das Ventil 238 beispielsweise mit der Steuereinrichtung verbunden werden. Das Ventil 238 kann händisch, pneumatisch oder elektrisch über die Steuereinrichtung betrieben werden. Die Figuren 1 und 2 zeigen jeweils Ventileinsteller 250, mit Hilfe der Ventile 238 eingestellt werden können. Diese können bereits vormontiert sein oder an die Verteilerplatte 200 anschließbar sein.

Zusätzlich kann die Verteilerplatte 200 zumindest einen Zugabeanschluss 252 aufweisen, über den ein weiteres Medium dem Fluidstrom 14 in der Verteilerplatte 200 zugeführt werden kann. Der Zugabeanschluss 252 kann als Anschlussstutzen und/oder als Sterilkonnektor ausgebildet sein. Grundsätzlich kann der Zugabeanschluss 252 an jeder beliebigen Position an der Verteilerplatte 200 angeordnet sein, wo die Zugabe eines Mediums in den Fluidstrom 14 gewünscht ist.

Wie in Figur 3 gezeigt, kann der Zugabeanschluss 252 derart positioniert sein, dass eine Zugabe eines Mediums in das Retentat ermöglicht wird. Hierzu kann der Zugabeanschluss 252 auf der Oberseite der Verteilerplatte 200 angeordnet sein und mit der Retentat-Abführleitung 242 verbunden sein. Vorzugsweise ist der Zugabeanschluss 252 vor dem Retentat-Ausgangsanschluss 240 bezüglich der Strömungsrichtung angeordnet. Wie in Figur 3 gezeigt, kann der Zugabeanschluss 252 ein Ventil 238, wie oben beschrieben, aufweisen. Entgegen der obigen Beschreibung ist es weiterhin möglich, dass der Retentat-Ausgangsanschluss 240 und der Zugabeanschluss 252 vertauscht sind.

Beispielsweise kann über den Zugabeanschluss 252 in Figur 3 ein Diafiltrationsmedium und/oder ein Puffer dem Retentat zugeführt werden.

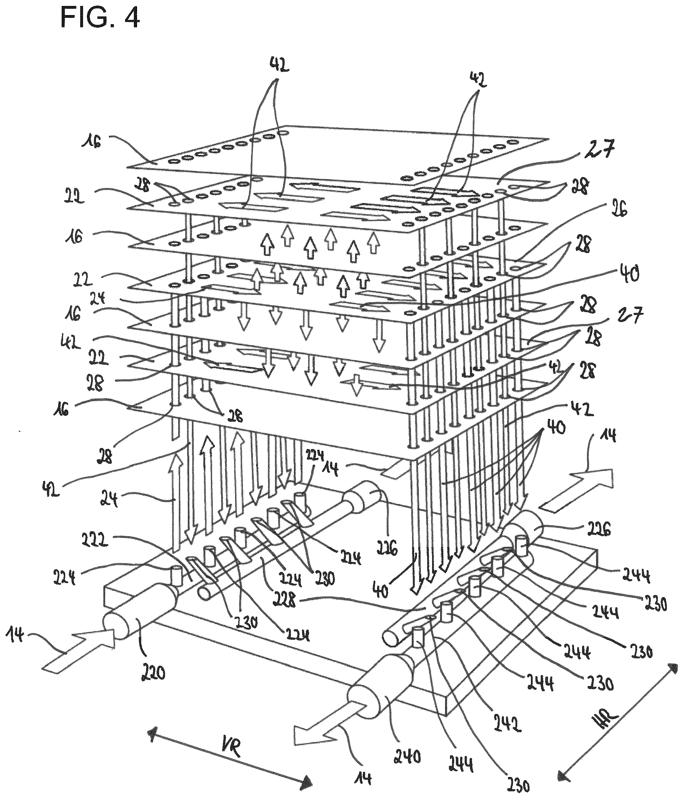

**Figur 4** zeigt exemplarisch eine Filtereinheit 10 zur Crossflowfiltration, welche in dem oben beschriebenen Filtersystem 100 einsetzbar ist. Es ist jedoch jede andere Filtereinheit 10 für das Filtersystem 100 verwendbar, sofern der Fluidstrom 14 durch die Filtereinheit 10 mit der Verteilerplatte 200 kompatibel ist.

Die Explosionsansicht in Figur 4 zeigt in der untersten Ebene schematisch die Zufuhr- und Abfuhrleitungen der Verteilerplatte 200, über die die Filtereinheit 10 gespeist wird und das Retentat und Permeat abgeführt werden kann. Jegliche Informationen zu diesen Leitungen, die bereits oben zur Verteilerplatte 200 gegeben wurden, gelten somit entsprechend für Figur 4. Um zu verdeutlichen, wie die Filtereinheit 10 im montierten Zustand an der Verteilerplatte 200 angeordnet ist, sind die horizontale Richtung HR und vertikale Richtung VR in Figur 4 eingezeichnet.

Die Filtereinheit 10 weist vorzugsweise zumindest ein Filtermedium 16 auf, das zumindest ein poröses Material umfasst, das danach gewählt bzw. verwendet wird, welche Partikel bzw. welche Stoffe mit Hilfe der Filtereinheit 10 aus dem Fluidstrom 14 herausgefiltert werden soll. Beispielsweise kann es sich bei dem Filtermedium 16 um einen Virusfilter, einen Sterilfilter, einen Tiefenfilter oder einen Membranadsorber handeln.

In Figur 4 weist die Filtereinheit 10 mehrere Schichten eines Filtermediums 16 auf, die aufeinander gestapelt sind und vorzugsweise durch im Wesentlichen durchlässige (insbesondere netz- bzw. gewebeartige) Abstandshalter 22 voneinander beabstandet sind.

Sowohl die Filtermedien 16 als auch die Abstandshalter 22 weisen auf gegenüberliegenden Seiten jeweils Durchgangsbohrungen 28 auf, welche entlang der horizontalen Richtung HR angeordnet sind. Sind die Filtermedien 16 und die Abstandshalter 22 aufeinander gestapelt, bilden die Durchgangsbohrungen 28, die sich überlappen, jeweils einen Kanal. Jeder dieser Kanäle ist dazu ausgelegt, einen Fluidstrom 14 zu führen. Hierzu stehen die Kanäle jeweils mit entweder der Zuführleitung 222, einer der Permeat-Abführleitung 228 oder der Retentat-Abführleitung 242 in Verbindung.

Kanäle, die mit der Zuführleitung 222 in Verbindung stehen, sind dazu ausgelegt einen Zuführstrom 24 zu führen. Der Zuführstrom 24 umfasst dabei das zu filtrierende Medium. Der Zuführstrom 24 wird bis zu einem Abstandshalter 22 geführt, welcher zwischen zwei Filtermedien 16 angeordnet ist. Dieser wird hier als Zuführ-Abstandshalter 26 bezeichnet. Der Zuführstrom 24 strömt anschließend zwischen einer Oberseite des Zuführ-Abstandshalters 26 und einem Filtermedium 16 und einer Unterseite des Zuführ-Abstandshalters 26 und einem Filtermedium 16.

Das Filtermedium 16 ist fluidisch durchlässig, wobei filtermediumspezifische Stoffe das Filtermedium 16 nicht passieren können. Filtermediumspezifische Stoffe, welche das Filtermedium 16 nicht passieren können, werden als Retentat aus der Filtereinheit 10 abtransportiert. Der Anteil des Zuführstroms 24 jedoch, der das Filtermedium 16 passieren kann, wird als Permeat aus der Filtereinheit 10 abtransportiert.

Da der Zuführstrom 24 entlang der Filtermedien 16 strömt, kann ein Teil des Zuführstroms 24 das Filtermedium 16 durchdringen. Der andere Teil des Zuführstroms 24, der das Filtermedium 16 nicht durchdringen kann (Retentat-Abführstrom 40), strömt weiter an dem Zuführ-Abstandshalter 26 zur gegenüberliegenden Seite des Zuführ-Abstandshalters 26. Dort tritt es in entsprechende Kanäle ein, die dazu ausgelegt sind, den Retentat-Abführstrom 40 zu einer Retentat-Abführleitung 242 zu führen.

Der Anteil des Zuführstroms 24, der jeweils ein Filtermedium 16 passieren kann, strömt anschließend auf die gegenüberliegende Seite des Filtermediums 16, das er passiert hat. Dieser Anteil des Zuführstroms 24 wird nun als Permeat-Abführstrom 42 bezeichnet. Dieser strömt zwischen dem Filtermedium 16 und einem Abstandshalter 22 zu den gegenüberliegenden Seiten des Abstandshalters 22 in Kanäle, die dazu ausgelegt sind, den Permeat-Abführstrom 42 zu der jeweiligen Permeat-Abführleitung 228 zu führen. Dieser Abstandshalter 22 wird hier als Permeat-Abstandshalter 27 bezeichnet.

Die oben beschriebene, gewünschte Fluidführung in den jeweiligen Kanälen bzw. Fluidverbindungen kann z.B. durch selektives Abdichten mittels Dichtelemente (z.B. Silikonringe) entsprechender Öffnungen der Durchgangsbohrungen 28 zu den Zwischenräumen zwischen dem Filtermedium 16 und dem Abstandshalter 22 (d.h. Zuführ-Abstandshalter 26 oder Permeat-Abstandshalter 27) erhalten werden.

Im montierten Zustand des Filtersystems 100 sind die Kanäle der Filtereinheit 10, welche benachbart zu der Abschlussplatte 204 angeordnet ist, vorzugsweise fluidisch nach außen verschlossen. Beispielsweise können die Kanäle verklebt oder durch Spritzguss verschlossen sein. Alternativ kann eine Plastikplatte, welche zwischen Abschlussplatte 204 und Filtereinheit 10, welche benachbart zu der Abschlussplatte 204 angeordnet ist und welche mit der Filtereinheit 10 beispielsweise verklebt ist, die Kanäle verschließen. Durch ein Verschließen der Kanäle kann verhindert werden, dass die Abschlussplatte 204 durch das Fluid kontaminiert wird. Hierdurch kann gewährleistet werden, dass die Abschlussplatte 204 wiederverwendet werden kann.

Das beschriebene Filtersystem 100 kann in vorteilhafter Weise als Einheit zur an den Benutzer geliefert werden, um mit dessen bestehendem System verbunden zu werden. Vorzugsweise ist das Filtersystem 100 bereits vormontiert und sterilisiert, so dass der Benutzer das Filtersystem 100 lediglich mit wenigen Handgriffen in sein System einsetzen muss. Das Filtersystem 100 selbst benötigt keine Schläuche, so dass der Einsatz des Filtersystems 100 für den Benutzer sehr vereinfacht wird.

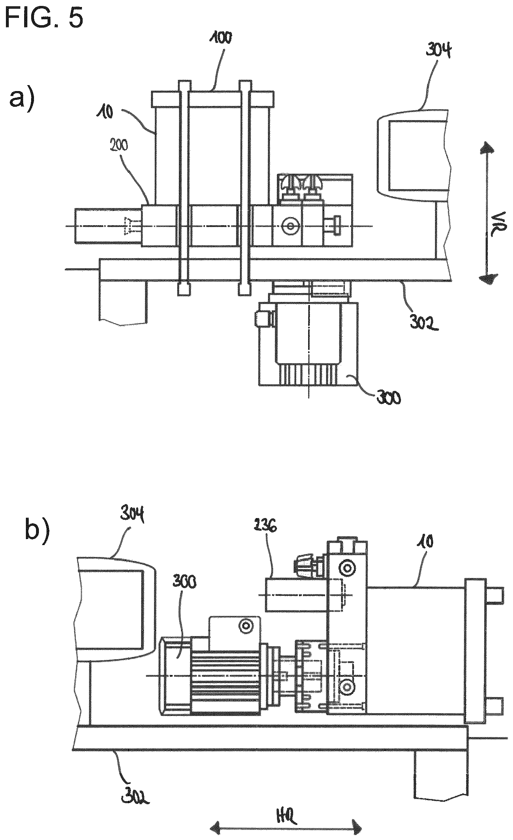

Die Figuren 5a) und5b) zeigen jeweils einen Arbeitstisch 302, an dem das Filtersystem 100 montierbar ist. An dem Arbeitstisch 302 kann beispielsweise auch die oben beschriebene Steuereinrichtung 304 angeordnet sein. Aufgrund der konstruktiven Ausgestaltung des Filtersystems 100 kann dieses sowohl in vertikaler Ausrichtung (siehe Figur 5a)) als auch in horizontaler Ausrichtung (siehe Figur 5b)) an dem Arbeitstisch 302 befestigt werden.

Im Falle einer vertikalen Ausrichtung können die Halteelemente 206 dazu verwendet werden, das Filtersystem 100 an dem Arbeitstisch 302 zu montieren. Im konkreten Fall der Figur 5a), könnten die Haltestangen 208 derart über die Verteilerplatte 200 hinausragen, dass diese auch den Arbeitstisch 302 durchdringen. Die Abschlussmuttern 210 sichern das Filtersystem 100 an dem Arbeitstisch 302.

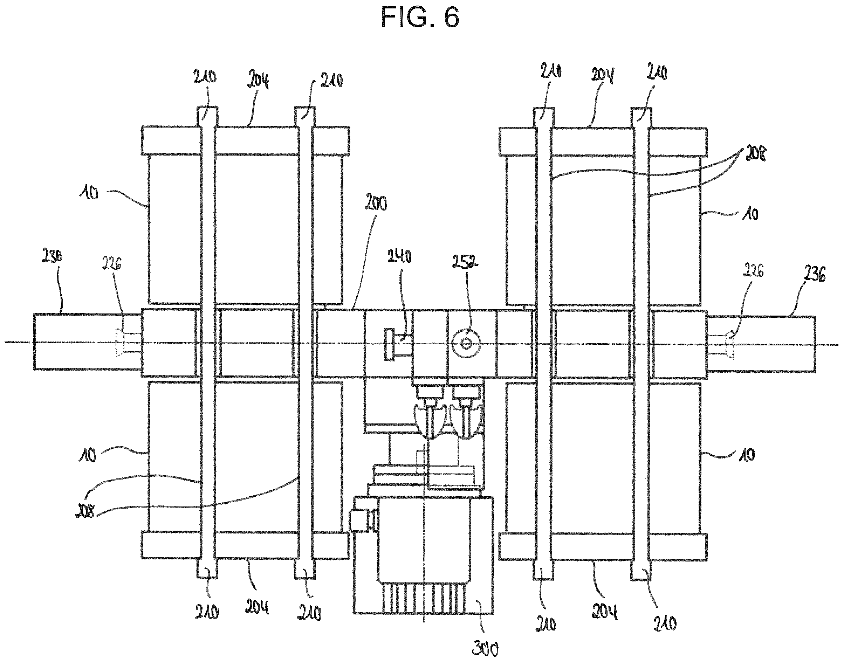

**Figur 6** zeigt eine weitere Ausführungsform eines Filtersystems 100. Das Filtersystem 100 unterscheidet sich hier von den bereits beschriebenen Filtersystemen 100 darin, dass die Verteilerplatte 200 derart ausgelegt ist, dass die Filterfläche um zumindest zwei weitere Filtereinheiten 10 erweitert werden kann.

Das gezeigte Filtersystem 100 entspricht einem Filtersystem 100 nach Figur 3, wobei der Verteilerplatte 200 nach links erweitert wird, um an der Erweiterung der Verteilerplatte 200 auf entgegengesetzten Seiten jeweils ebenfalls zumindest eine Filtereinheit 10 anordnen zu können. Es ist jedoch auch möglich, dass nur auf einer Seite der Erweiterung der Verteilerplatte 200 zumindest eine Filtereinheit 10 angeordnet ist. Beschreibungen zu den Figuren 1 und 2 gelten hier entsprechend.

Mit anderen Worten befindet sich hier an der Verteilerplatte 200 mittig die Pumpe und nach links und rechts erstreckt sich die Verteilerplatte 200. An beiden Verteilerplattenhälften ist jeweils an entgegengesetzten Seiten der Verteilerplatte 200 jeweils zumindest eine Filtereinheit 10 angeordnet. An entgegengesetzten Enden der Verteilerplatte 200 befinden sich hier die Permeat-Ausgangsanschlüsse 226, während sich mittig, vorzugsweise auf Höhe der Pumpe, der Retentat-Ausgangsanschluss 240 und der Eingangsanschluss 220 befindet. Erklärungen zur Fluidführung innerhalb der Verteilerplatte 200 bezüglich der Figuren 1 und 2 gelten hier entsprechend.

Alternativ können zwei Verteilerplatte 200 miteinander gekoppelt werden, um die in Figur 6 gezeigt Systemerweiterung bereitzustellen.

Das Filtersystem 100 der vorliegenden Erfindung bietet somit ein einfaches und kostengünstiges System, das leicht in der Handhabung ist und keinerlei Schläuche genötigt. Je nach Bedarf kann außerdem die Filterfläche in einfacher Weise erweitert werden.

Die vorliegende Anmeldung kann sich außerdem auf einen der folgenden Items beziehen:
Item 1. Filtersystem (100) für biopharmazeutische Prozesse umfassend:
   - zumindest eine Filtereinheit (10); und
   - zumindest eine Verteilerplatte (200), an welcher die zumindest eine Filtereinheit (10) anliegt und welche eine Verteilereinheit (202) umfasst, welche innerhalb der Verteilerplatte (200) angeordnet ist und fluidisch mit der zumindest einen Filtereinheit (10) verbunden ist;
      wobei die Verteilereinheit (202) dazu ausgelegt ist, das zu filtrierende Fluid zu der zumindest einen Filtereinheit (10) zu führen und/oder das filtrierte Fluid von der zumindest einen Filtereinheit (10) aufzunehmen und abzuführen, und
      wobei die Verteilereinheit (202) zumindest ein aktives Steuerelement (232; 238) umfasst, mittels dem ein Fluidstrom (14), welcher durch die Verteilereinheit (202) und die zumindest eine Filtereinheit (10) strömt, steuerbar oder regelbar ist,
      wobei die Verteilereinheit (202) schlauchlos ist, und
      wobei die Fluidverbindung zwischen der zumindest einen Filtereinheit (10) und der Verteilerplatte (200) schlauchlos ist.
Item 2. Filtersystem (100) nach Item 1, wobei die Verteilereinheit (202) ein Rohrleitungssystem (218) umfasst, welches sich innerhalb der Verteilerplatte (200) erstreckt und dazu ausgelegt ist, das zu filtrierende Fluid und/oder das filtrierte Fluid zu führen.
Item 3. Filtersystem (100) nach Item 1 oder 2, wobei das aktive Steuerelement zumindest einen Pumpenkopf (232) umfasst, der mit einem Pumpenantrieb (300) verbindbar ist, um das Fluid durch die Verteilerplatte (200) und die zumindest eine Filtereinheit (10) zu pumpen.
Item 4. Filtersystem (100) nach Item 2 oder 3, wobei das aktive Steuerelement zumindest ein Ventil (238) umfasst, welches dazu ausgelegt ist, den Fluidstrom (14) durch die Verteilerplatte (200) und/oder die zumindest eine Filtereinheit (10) zu regeln oder zu steuern.
Item 5. Filtersystem (100) nach einem der vorangehenden Items, wobei die Verteilereinheit (202) zumindest einen Sensor (236) umfasst, welcher dazu ausgelegt ist, zumindest einen Parameter des Fluidstroms (14) innerhalb der Verteilereinheit (202) zu messen.
Item 6. Filtersystem (100) nach einem der vorangehenden Items, ferner umfassend zumindest eine Abschlussplatte (204), welche mittels zumindest einem Halteelement (206) mit der Verteilerplatte (200) verbunden ist,
   wobei die Filtereinheit (10) zwischen der Verteilerplatte (200) und der Abschlussplatte (204) angeordnet ist und durch die Abschlussplatte (204) an der Verteilerplatte (200) gehalten wird.
Item 7. Filtersystem (100) nach einem der vorangehenden Items, wobei die Verteilerplatte (200) aus Kunststoff gefertigt ist.
Item 8. Filtersystem (100) nach einem der vorangehenden Items, wobei die zumindest eine Filtereinheit (10) und/oder die Verteilerplatte (200) durch Gamma-Bestrahlung, Begasung und/oder Autoklavieren sterilisierbar ist.
Item 9. Filtersystem (100) nach einem der vorangehenden Items, umfassend zumindest eine erste Filtereinheit (214) und zumindest eine zweite Filtereinheit (216), wobei die zumindest eine erste (214) und die zumindest eine zweite Filtereinheit (216) auf entgegengesetzten Seiten der Verteilerplatte (200) anliegen.
Item 10. Filtersystem (100) nach einem der vorangehenden Items, wobei die Filtereinheit (10) und die Verteilerplatte (200) miteinander verklebt sind oder aus einem Stück spritzgegossen sind.
Item 11. Verteilerplatte (200) für ein Filtersystem (100) für biopharmazeutische Prozesse umfassend eine Verteilereinheit (202), welche innerhalb der Verteilerplatte (200) angeordnet ist und fluidisch mit zumindest einer Filtereinheit (10) verbindbar ist, indem die Filtereinheit (10) an der Verteilerplatte (200) anliegt;
   wobei die Verteilereinheit (202) dazu ausgelegt ist, das zu filtrierende Fluid zu der zumindest einen Filtereinheit (10) zu führen und/oder das filtrierte Fluid von der zumindest einen Filtereinheit (10) aufzunehmen und abzuführen, und
   wobei die Verteilereinheit (202) zumindest ein aktives Steuerelement (232; 238) umfasst, mittels dem ein Fluidstrom (14), welcher durch die Verteilereinheit (202) und die zumindest eine Filtereinheit (10) strömt, steuerbar oder regelbar ist,
   wobei die Verteilereinheit (202) schlauchlos ist, und
   wobei die Fluidverbindung zwischen der zumindest einen Filtereinheit (10) und der Verteilerplatte (200) schlauchlos ist.
Item 12. Verfahren zum Herstellen eines Filtersystems (100) für biopharmazeutische Prozesse umfassend die Schritte:
   - Bereitstellen zumindest einer Filtereinheit (10); und
   - Bereitstellen zumindest einer Verteilerplatte (200), welche eine schlauchlose Verteilereinheit (202), welche innerhalb der Verteilerplatte (200) angeordnet ist, und zumindest ein aktives Steuerelement (232; 238) umfasst; und
   - Schlauchloses Anordnen der zumindest einen Filtereinheit (10) an der Verteilerplatte (200), derart dass die zumindest eine Filtereinheit (10) und die Verteilereinheit (202) fluidisch miteinander verbunden sind;
      wobei die Verteilereinheit (202) dazu ausgelegt ist, das zu filtrierende Fluid zu der zumindest einen Filtereinheit (10) zu führen und/oder das filtrierte Fluid von der zumindest einen Filtereinheit (10) aufzunehmen und abzuführen, und
      wobei das zumindest eine aktive Steuerelement (232; 238) dazu ausgelegt ist, einen Fluidstrom (14), welcher durch die Verteilereinheit (202) und die zumindest eine Filtereinheit (10) strömt, zu steuern oder zu regeln.

### Bezugszeichenliste

- 10: Filtereinheit
- 14: Fluidstrom
- 16: Filtermedium
- 22: Abstandshalter
- 24: Zuführstrom
- 26: Zuführ-Abstandshalter
- 27: Permeat-Abstandshalter
- 28: Durchgangsbohrung
- 40: Retentat-Abführstrom
- 42: Permeat-Abführstrom

- 100: Filtersystem

- 200: Verteilerplatte
- 202: Verteilereinheit
- 204: Abschlussplatte
- 206: Halteelement
- 208: Haltestange
- 210: Abschlussmutter
- 212: Kerbe
- 214: Erste Filtereinheit
- 216: Zweite Filtereinheit
- 218: Rohrleitungssystem
- 220: Eingangsanschluss
- 222: Zuführungsleitung
- 224: Einströmöffnung
- 226: Permeat-Ausgangsanschluss
- 228: Permeat-Abführleitung
- 230: Permeat-Abführöffnung
- 232: Pumpenkopf (aktives Steuerelement)
- 236: Sensor
- 238: Ventil (aktives Steuerelement)
- 240: Retentat-Ausgangsanschluss
- 242: Retentat-Abführleitung
- 244: Retentat-Abführöffnung
- 248: Anschlussausnehmung
- 250: Ventileinsteller
- 252: Zugabeanschluss

- 300: Pumpenantrieb
- 302: Arbeitstisch
- 304: Steuereinrichtung

- HR: Horizontale Richtung
- VR: Vertikale Richtung

## Patentansprüche

1. Filtersystem (100) für biopharmazeutische Prozesse umfassend:
- zumindest eine Filtereinheit (10); und
- zumindest eine Verteilerplatte (200), an welcher die zumindest eine Filtereinheit (10) anliegt und welche eine Verteilereinheit (202) umfasst, welche innerhalb der Verteilerplatte (200) angeordnet ist und fluidisch mit der zumindest einen Filtereinheit (10) verbunden ist;
wobei die Verteilereinheit (202) ein Rohrleitungssystem (218) umfasst, welches sich innerhalb der Verteilerplatte (200) erstreckt und derart ausgebildet ist, dass das zu filtrierende Fluid über einen Eingangsanschluss (220) der zumindest einen Filtereinheit (10) zugeführt werden kann und/oder das filtrierte Fluid von der zumindest einen Filtereinheit (10) aufgenommen und abgeführt werden kann, und
wobei die Verteilereinheit (202) zumindest ein aktives Steuerelement (232; 238) umfasst, welches ausgebildet ist, einen Fluidstrom (14), welcher durch die Verteilereinheit (202) und die zumindest eine Filtereinheit (10) strömt, zu steuern oder zu regeln,
wobei die Verteilereinheit (202) schlauchlos ist,
wobei die Fluidverbindung zwischen der zumindest einen Filtereinheit (10) und der Verteilerplatte (200) schlauchlos ist, und
wobei das Rohrleitungssystem (218) zwei Ausganganschlüsse (226) aufweist, über die das filtrierte Fluid aus der Verteilerplatte (200) abgeführt werden kann.

2. Filtersystem (100) nach Anspruch 1, wobei die Ausgangsanschlüsse (226) jeweils mit einer Permeat-Abführleitung (228) verbunden sind,
wobei die Permeat-Abführleitung (228) mit zumindest einer Permeat-Abführöffnung (230) verbunden ist, über die das Permeat aus der zumindest einen Filtereinheit (10) in die Permeat-Abführleitung (228) einströmen kann.

3. Filtersystem (100) nach Anspruch 2, wobei mehrere Permeat-Abführöffnungen (230) entlang der einzelnen Permeat-Abführleitungen (228) angeordnet sind.

4. Filtersystem (100) nach Anspruch 2 oder 3, wobei die Permeat-Abführöffnungen (230) einen kreisrunden, ovalen oder schlitzförmigen Querschnitt aufweisen.

5. Filtersystem (100) nach einem der vorangehenden Ansprüche, wobei die Ausgangsanschlüsse (226) an einer Seite der Verteilerplatte (200) angeordnet sind, welche der Seite entgegengesetzt ist, an der der Eingangsanschluss (220) angeordnet ist.

6. Filtersystem (100) nach einem der vorangehenden Ansprüche, wobei das aktive Steuerelement in die Verteilerplatte (200) integriert ist.

7. Filtersystem (100) nach einem der vorangehenden Ansprüche, wobei das aktive Steuerelement zumindest ein Ventil (238) umfasst, welches dazu ausgelegt ist, den Fluidstrom (14) durch die Verteilerplatte (200) und/oder die zumindest eine Filtereinheit (10) zu regeln oder zu steuern.

8. Filtersystem (100) nach einem der vorangehenden Ansprüche, wobei die Verteilereinheit (202) zumindest einen Sensor (236) umfasst, welcher dazu ausgelegt ist, zumindest einen Parameter des Fluidstroms (14) innerhalb der Verteilereinheit (202) zu messen.

9. Filtersystem (100) nach einem der vorangehenden Ansprüche, ferner umfassend zumindest eine Abschlussplatte (204), welche mittels zumindest einem Halteelement (206) mit der Verteilerplatte (200) verbunden ist,
wobei die Filtereinheit (10) zwischen der Verteilerplatte (200) und der Abschlussplatte (204) angeordnet ist und durch die Abschlussplatte (204) an der Verteilerplatte (200) gehalten wird.

10. Filtersystem (100) nach einem der vorangehenden Ansprüche, wobei die Verteilerplatte (200) aus Kunststoff gefertigt ist.

11. Filtersystem (100) nach einem der vorangehenden Ansprüche, wobei die zumindest eine Filtereinheit (10) und/oder die Verteilerplatte (200) durch Gamma-Bestrahlung, Begasung und/oder Autoklavieren sterilisierbar ist.

12. Filtersystem (100) nach einem der vorangehenden Ansprüche, umfassend zumindest eine erste Filtereinheit (214) und zumindest eine zweite Filtereinheit (216), wobei die zumindest eine erste (214) und die zumindest eine zweite Filtereinheit (216) auf entgegengesetzten Seiten der Verteilerplatte (200) anliegen.

13. Filtersystem (100) nach einem der vorangehenden Ansprüche, wobei die Filtereinheit (10) und die Verteilerplatte (200) miteinander verklebt sind oder aus einem Stück spritzgegossen sind.

14. Verteilerplatte (200) für ein Filtersystem (100) für biopharmazeutische Prozesse umfassend eine Verteilereinheit (202), welche innerhalb der Verteilerplatte (200) angeordnet ist und fluidisch mit zumindest einer Filtereinheit (10) verbindbar ist, indem die Filtereinheit (10) an der Verteilerplatte (200) anliegt;
wobei die Verteilereinheit (202) ein Rohrleitungssystem (218) umfasst, welches sich innerhalb der Verteilerplatte (200) erstreckt und derart ausgebildet ist, dass das zu filtrierende Fluid über einen Eingangsanschluss (220) der zumindest einen Filtereinheit (10) zugeführt werden kann und/oder das filtrierte Fluid von der zumindest einen Filtereinheit (10) aufgenommen und abgeführt werden kann, und
wobei die Verteilereinheit (202) zumindest ein aktives Steuerelement (232; 238) umfasst, welches ausgebildet ist, einen Fluidstrom (14), welcher durch die Verteilereinheit (202) und die zumindest eine Filtereinheit (10) strömt, zu steuern oder zu regeln,
wobei die Verteilereinheit (202) schlauchlos ist,
wobei die Fluidverbindung zwischen der zumindest einen Filtereinheit (10) und der Verteilerplatte (200) schlauchlos ist, und
wobei das Rohrleitungssystem (218) zwei Ausganganschlüsse (226) aufweist, über die das filtrierte Fluid aus der Verteilerplatte (200) abgeführt werden kann.

15. Verfahren zum Herstellen eines Filtersystems (100) für biopharmazeutische Prozesse umfassend die Schritte:
- Bereitstellen zumindest einer Filtereinheit (10); und
- Bereitstellen zumindest einer Verteilerplatte (200), welche eine schlauchlose Verteilereinheit (202), welche innerhalb der Verteilerplatte (200) angeordnet ist, und zumindest ein aktives Steuerelement (232; 238) umfasst; und
- Schlauchloses Anordnen der zumindest einen Filtereinheit (10) an der Verteilerplatte (200), derart dass die zumindest eine Filtereinheit (10) und die Verteilereinheit (202) fluidisch miteinander verbunden sind;
wobei die Verteilereinheit (202) ein Rohrleitungssystem (218) umfasst, welches sich innerhalb der Verteilerplatte (200) erstreckt und derart ausgebildet ist, dass das zu filtrierende Fluid über einen Eingangsanschluss (220) der zumindest einen Filtereinheit (10) zugeführt werden kann und/oder das filtrierte Fluid von der zumindest einen Filtereinheit (10) aufgenommen und abgeführt werden kann, und
wobei das zumindest eine aktive Steuerelement (232; 238) dazu ausgelegt ist, einen Fluidstrom (14), welcher durch die Verteilereinheit (202) und die zumindest eine Filtereinheit (10) strömt, zu steuern oder zu regeln, und
wobei das Rohrleitungssystem (218) zwei Ausganganschlüsse (226) aufweist, über die das filtrierte Fluid aus der Verteilerplatte (200) abgeführt werden kann.
